# EUROPEAN PATENT APPLICATION

(11) **EP 2 574 909 A1**
(43) Date of publication of application: **03.04.2013**
(21) Application number: 11183228.3
(22) Date of filing: 29.09.2011
(51) Int. Cl.: G01N 27/38

(54) **Electrode sensor with automatic cell sand dosing**

(71) Applicant: Siemens Aktiengesellschaft, 80333 München (DE)
(72) Inventor: Zeh, Thomas, 89233 Neu-Ulm (DE)

(57) **Abstract**

The present invention relates to an electrode sensor arrangement (1).

This electrode sensor arrangement (1) comprises a cell body (2) with a measurement electrode (3) to be arranged in said cell body (2) for measuring a physical parameter of a fluid (5) flowing through said cell body (2). A cell sand dosage tank (6) is connected to said cell body (2) using a cell sand dosing means (7) for automatically dosing cell sand (9) out of said cell sand dosage tank (6) into said cell body (2). An electronic device (8) is connected to said cell sand dosing means (7) for controlling said automatically dosing of cell sand (9) into said cell body (2).

## Description

### TECHNICAL FIELD OF THE INVENTION AND PRIOR ART

The present invention relates to an electrode sensor arrangement with an electrode to be cleaned with cell sand (hydrodynamic cell sand cleaning).

A sensor arrangement with a measurement electrode, following referred as electrode sensor arrangement or just as electrode sensor, for example a chlorine used for a measurement of (free) chlorine is known from "Betriebsanleitung - Wallace & Tiernan® Mess- und Regelsystem SFC (06/2011)" or "Betriebsanleitung - Wallace & Tiernan® Mess-, Regel- und Dosiersystem PCS *plus* (06/2010)" , Siemens AG, 89312 Günzburg " as well as "Einbau und Betriebsanleitung - JUDO LIBELLE Doscon JLD-E", Judo Wasseraufbereitung (2009/2010).

These SFC, PCS plus or JLD-E chlorine electrode sensor comprises - as a pressurized as well as an unpressurized/pressureless version - a flow block assembly/module with a chlorine sensor measuring electrode arranged in a cell body located in said flow block assembly and being flushed with (sample) water to be controlled and an electronic module connected with said chlorine sensor measuring electrode by wires controlling and operating the measurement of the (free) chlorine.

The flow block module, i.e. the cell body arranged in the flow block module, will be flowed through/flushed by the (sample) water with said water flowing into the flow block module, i.e. the cell body, at a (sample) water inlet and flowing out of the flow block module, i.e. the cell body, at a (sample) water outlet.

The (sample) water inlet - in case of the pressureless version of the SFC, PCS plus or JLD-E chlorine electrode sensor as well as in case of the pressurized version - is equipped with a (ball) valve as well as the (sample) water outlet - only in case of the pressurized version - is also equipped with a (ball) valve, said (ball) valves could be closed and opened manually while controlling the (sample) water entering/leaving the flow block module, i.e. the cell body.

The measuring electrode will be cleaned hydrodynamicly by cell sand (hydrodynamic cell sand cleaning), for example silica sand or corundum, filled in the cell body and grinding the measuring electrode while the measuring electrode is flushed by the (sample) water flowing through the cell body.

A pour hole - covered by a protection plug - is arranged at a cover of the cell body for pouring the cell sand into the cell body.

A drain with a drain screw is arranged at an end of the cell body for draining off the (sample) water and the cell sand out of the cell body.

The cell sand required for the hydrodynamic (cell sand) cleaning of the chlorine sensor measuring electrode grinds itself down over time until the cell sand becomes very fine. An efficiency of the hydrodynamic cleaning will decrease while the cell sand becomes fine - and, consequently, the (free) chlorine measurement would no longer be reliable.

The cell sand required for the hydrodynamic (cell sand) cleaning of the chlorine sensor measuring electrode also will be flushed out by the (sample) water flowing through the cell body gradually with insufficient cell sand causing insufficient cleaning of the measuring electrode and unreliable (free) chlorine measurement.

Therefore, the cell sand has to be controlled continuously and refilled and/or replaced, for example quarterly or half-yearly.

This cell sand refill/-placement procedure must be performed manually by a (maintenance) operator in a difficult and complex multi-step procedure which is described - exemplarily for the pressurized version of the sensor arrangement - in the following.

The (ball) valves of the (sample) water inlet and outlet of the flow block assembly, i.e. cell body, have to be closed.

The drain screw has to been opened carefully as the pressure in the cell body has to be released as well as the cell body, i.e. the (sample) water in the cell body as well as the fully worn cell sand, will be drained. After draining the cell body the drain screw has to been fixed again.

A protection plug covering the pour hole at the cover of the cell body has to be removed.

New cell sand - provided in a cell sand plastic bottle - has to be filled/poured out of the cell sand plastic bottle into the cell body - via the pour hole - in a defined amount while a cap of the cell sand plastic bottle could serve for measuring the amount of new cell sand to be filled/poured in the cell body.

The pour hole at the cover of the cell body has to be cleaned by rinsing off with distilled water. The protection plug at the cover of the cell body has to be fixed again.

The (ball) valves of the (sample) water inlet and outlet of the flow block assembly, i.e. the cell body, could be opened again.

Therefore, operation and maintenance of a (free) chlorine electrode sensor are non-efficient, error-prone and cost-intensive.

### SUMMARY OF THE INVENTION

It is a first objective of the invention to provide an electrode sensor, especially a chlorine electrode sensor, by which the above-mentioned shortcomings can be mitigated.

It is a further objective of the invention to provide an electrode sensor which can operate more reliably and which can be maintained more efficiently, fail-safely and economically.

These objectives are according to the invention achieved by providing an electrode sensor arrangement.

This electrode sensor arrangement comprises a cell body with a measurement electrode to be arranged in said cell body for measuring a physical parameter of a fluid, for example (sample) water, flowing through said cell body.

Said cell body can - therefore - especially be equipped with a fluid inlet, furthermore said fluid inlet especially comprising an electromagnet (back pressure) fluid inlet valve, as well as a fluid outlet, also furthermore said fluid outlet especially - in case of pressurized electrode sensor arrangement - comprising an electromagnet (back pressure) fluid outlet valve, providing said fluid flowing into/out of said cell body.

This electrode sensor arrangement further comprises a cell sand dosage tank connected to said cell body using a cell sand dosing means, for example an electromagnetic valve, especially an electromagnetic back pressure valve, for automatically dosing cell sand out of said cell sand dosage tank into said cell body.

This electrode sensor arrangement also comprises an electronic device connected to said cell sand dosing means, for example said electromagnetic (back pressure) valve, for controlling said automatically dosing of cell sand into said cell body.

Controlling said automatically dosing of cell sand by said electronic device - while said electronic device being functionally connected to said cell sand dosing means - can mean that said electronic device is facilitated to control said - for example - cell sand dosing electromagnetic valve by switching said valve.

In other words - the invention relates to an automatic cell sand dosage for an electrode sensor arrangement replacing the manual operation for refilling and replacing the run-down cell sand.

The automatic cell sand dosage is supervised by said inventive electronic device, for example an electronic module (analysis electronic) comprising a processor/computer equipped with controlling/measuring software, controlling the cell sand dosing means, i.e. the electromagnetic valve.

An existing electrode sensor arrangement as well as an existing electronic module - both known in prior art - can further be used while said existing electrode sensor equipment can be equipped with said inventive cell sand dosage tank connected to said cell body by said cell sand dosing means as well as said existing electronic module can be further equipped with said controlling/measuring software facilitating the inventive automatic cell dosing - as well as - existing controlling/measuring software can be expended respectively facilitating the inventive automatic cell dosing.

While using said electronic device for controlling the automatic cell sand dosage, i.e. said automatically dosing, for example by switching said electromagnetic valve, i.e. said cell sand dosing means, the cell body can be filled with an exactly specified amount of new cell sand - therefore, saving costs.

A soiling of the electrode sensor arrangement, especially of threads in the cell body cover, for example threads for the measuring sensor to be fixed at the cell body cover, by manually handling the cell sand can be avoided. An additionally rinsing is not necessary any more.

The plastic bottle with the cell sand - used in prior art - can not be misplaced or be lost while the cell sand dosage tank is connected to said cell body.

Therefore, the invention provides a new, effective electrode sensor.

In a preferred embodiment said cell sand is quartz sand or aluminium oxide, especially fused aluminium oxide. These types of cell sand are approved and very effective for a hydrodynamic cleaning.

In a further preferred embodiment this electrode sensor arrangement further comprises a cell sand drain connected to said cell body using, i.e. by, a cell sand draining means, for example an electromagnetic valve, especially an electromagnetic back pressure valve, for automatically draining cell sand out of said cell body in said cell sand drain. The electronic device can be connected to said cell sand draining means for controlling said automatically draining of cell sand out of said cell body.

In another preferred embodiment said measurement electrode is a chlorine electrode measuring a concentration of a chlorine species dissolved in said fluid, especially measuring a free chlorine or chlorine dioxide equivalent in said fluid. Accordingly - said physical parameter to be measured can be a concentration of a chlorine species dissolved in said fluid, a free chlorine or chlorine dioxide equivalent in said fluid.

In a preferred embodiment said cell sand dosing means comprises a cell sand dosing valve, especially an electromagnetic (cell sand dosing) valve, furthermore especially a electromagnetic back pressure valve, being controlled by said electronic device.

Said electronic device can also be connected, for example by a wire, to said measurement electrode, said electronic device, i.e. said electronic module, also equipped for visualising and processing a measurement of said electrode and for controlling said measurement, said visualising and said processing.

In another preferred embodiment said electronic device is an electronic module, especially comprising a processor facilitating measuring/controlling software and/or further connectable to a computer facilitating measuring/controlling software.

In another preferred embodiment said cell body is arranged in a flow block assembly. Said flow block assembly can be of the pressurized or pressureless type.

Said flow block assembly can further be equipped with a fluid inlet, especially comprising an electromagnet (back pressure) fluid inlet valve, and a fluid outlet, especially - in case of the pressurized version - comprising an electromagnet (back pressure) fluid inlet valve or - in case of the presureless version - comprising a drain, for said fluid flowing into and out of said flow block assembly, i.e. flowing into and out of said cell body, said cell body being arranged in the flow block assembly.

Said electromagnet (back pressure) fluid inlet valve and/or said electromagnet (back pressure) fluid outlet valve can be functionally connected with said electronic device and controlled by said electronic device. Therefore, the flowing of said fluid flowing into/out of said flow block assembly, i.e. said cell body, can be controlled by said electronic device.

In a preferred embodiment said flow block assembly is a pressureless flow armature comprising said drain, wherein said fluid outlet is connected with said drain for said fluid flowing out of said flow block assembly.

In another preferred embodiment a cell sand storage tank is connected to said cell sand dosing tank providing said cell sand dosing tank with said cell sand.

Said cell sand dosage tank can be fixed at a cell body cover or can be mounted at a flow block module housing or wall with being connected to said cell body.

Functionality can be enhanced while a lightening means, especially a back lightening means, for example a LED or more LEDs, for lightening, especially for back lightening, said cell body is assembled to or installed in said electrode sensor arrangement.

At an electrode sensor arrangement - depending on pollution degree of said fluid by organic residues, for example skin shed, or inorganic residues, as iron or scale, - a cell body can be soiled and/or said pollution could be deposited at the cell body as well as the fluid can become turbidly. A view on a measuring electrode arranged in the cell body get worse as well as a visual check of a hydrodynamic cell sand cleaning could be made difficultly.

At said installation of said lightening means, for example a couple of LEDs arranged beside and/or rearward - relatively - to said cell body and/or in a upper region of said flow block module, said visuality to/on said cell body, i.e. said measuring electrode, can be improved and/or said visual check of said hydrodynamic cleaning can be enabled.

In a preferred embodiment said lightening means are a couple of LEDs arranged as said back lightening means at said cell body.

An electric supply for said LED can be realized by a (storage) battery operation and/or by said electronic device and/or by an installed water-power dynamo, especially arranged in a sample water inlet.

Software of the electronic device can be expanded by a display menu item "cell body backlight on/off", while said LED backlight can be turned on/off by an operator. Automatically backlight operations are also possible.

For an automatic cell sand refill/replacement operation said fluid flowing into said cell body can be stopped, for example by closing said electromagnetic fluid inlet valve via said electronic device. Said cell sand dosing means, for example said electromagnetic cell sand dosing valve, can be controlled - via said electronic device - for said automatically dosing said cell sand out of said cell sand dosage tank into said cell body - by an exactly defined amount of cell sand is dosed into said cell body. Said fluid flowing into said cell body can be restarted, for example by opening said electromagnetic fluid inlet valve via said electronic device.

Preferable, said cell body can be jetted with said fluid after a draining off of said cell sand out of said cell body and before said automatically dosing said cell sand out of said cell sand dosage tank into said cell body.

Further advantages as well as advantageous features of the invention appear from the following description.

### BRIEF DESCRIPTION OF THE DRAWINGS

With reference to the appended drawings, below follows specific descriptions of embodiments of the invention cited as examples.

In the drawing:
- Figure 1: is a schematic illustration of an electrode sensor arrangement measuring a chlorine concentration of (sample) water according to an embodiment of the invention and
- Figure 2: is a further schematic illustration of an electrode sensor arrangement measuring a chlorine concentration of (sample) water comprising a cell body backlight according to a further embodiment of the invention.

### DETAILED DESCRIPTION OF AN EMBODIMENT OF THE INVENTION

### AUTOMATIC CELL SAND DOSAGE

The present examples are directed to an electrode sensor arrangement 1 (electrode sensor 1) with an chlorine electrode 3 measuring a (free) chlorine concentration of (sample) water 5 - provided by a water circulation 26 - with said chlorine electrode 3 to be cleaned with cell sand 9 (hydrodynamic cell sand cleaning).

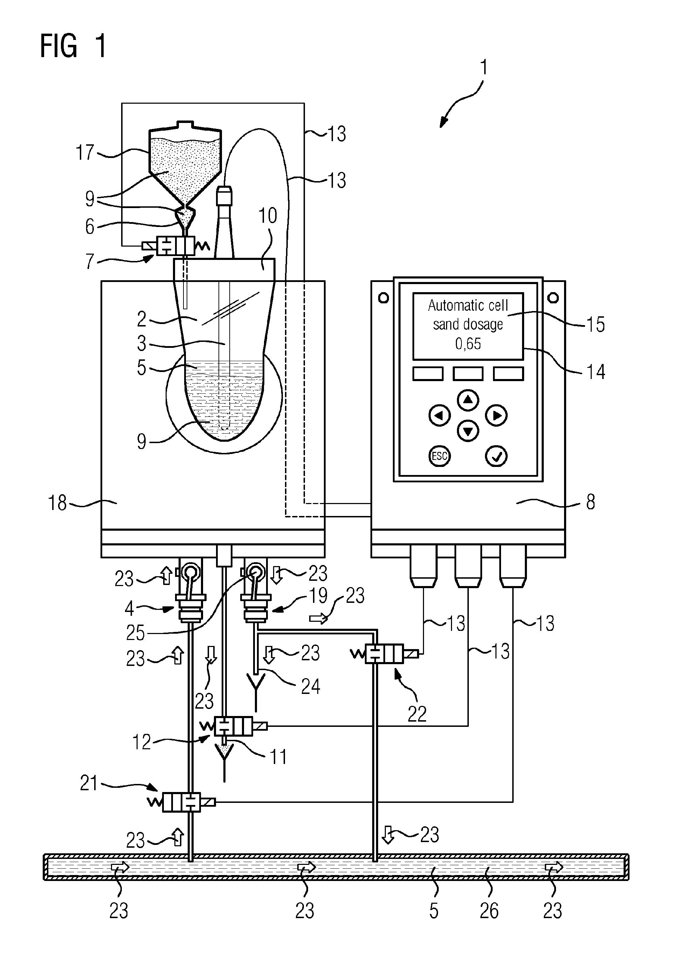

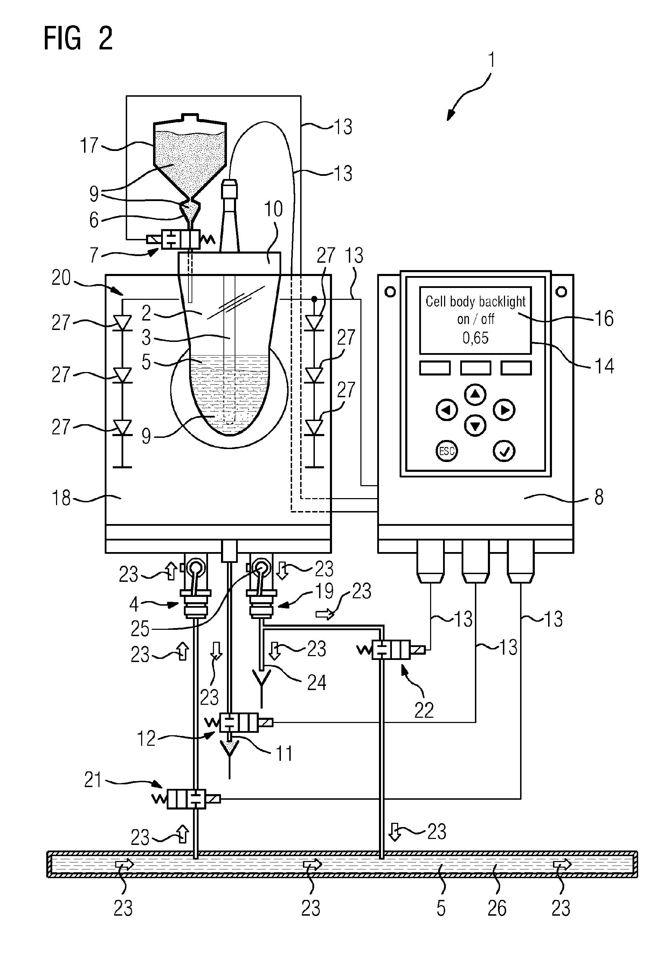

FIG 1 shows said electrode sensor 1 exemplary without a backlight (illumination) 20 whereas FIG 2 shows said electrode sensor 1 exemplary with the backlight (illumination) 20. Same reference numbers refers to same elements in the figures.

This electrode sensor 1 shown in FIG 1 and FIG 2 comprises - as a pressurized as well as an unpressurized/pressureless version - a flow block assembly/module 18 with said chlorine electrode 3 arranged in a cell body 2 located in said flow block assembly 18 and being flushed with said (sample) water 5 to be controlled, i.e. the chlorine concentration of said water 5 will be measured by said chlorine electrode 3.

This electrode sensor 1 shown in FIG 1 and FIG 2 further comprises - as a pressurized as well as an unpressurized/pressureless version - an electronic module 8 connected with said chlorine electrode 3 by a wire 13, said electronic module (analysing electronic) controlling and operating the measurement of the chlorine concentration of said water 5.

Said flow block module 18, i.e. said cell body 2 arranged in the flow block module 18, is be flowed through/flushed by said (sample) water 5 with said water 5 flowing into the flow block module 18, i.e. the cell body 2, at a (sample) water inlet 4 and flowing out of the flow block module 18, i.e. the cell body 2, at a (sample) water outlet 19.

The (sample) water inlet 4 - in case of the pressureless version of the electrode sensor 1 as well as in case of the pressurized version 1 - is equipped with an electromagnetic back pressure (water inlet) valve 21 as well as the (sample) water outlet 19 - only in case of the pressurized version - is also equipped with an electromagnetic back pressure (water outlet) valve 22, said valves 21, 22 being controlled (opened/closed) by said electronic module 8 while being connected to said electronic module 8 by wires 13.

The (sample) water inlet 4 - in case of the pressureless version of the electrode sensor 1 as well as in case of the pressurized version 1 - as well as the (sample) water outlet 19 - only in case of the pressurized version - are connected to said water circulation 26 with water 5 flowing there 23 to be controlled by said electrode sensor 1.

The water 5 of said water circulation 26 can flow 23 into said flow block assembly 18, i.e. into said cell body 2, via the (sample) water inlet 4 while said (sample) water 5 can flow out of the cell body 2, i.e. said flow block assembly 18, into said water circulation 26 via the (sample) water outlet 19.

In case of the pressureless version - the (sample) water outlet 19 is equipped - instead of being connected with the water circulation 26 - with a drain 24 for the (sample) water 5. A ball valve 25 of said (sample) water outlet 5 can be closed and opened manually for draining off the (sample) water 5 out of the cell body 2, i.e. said flow block assembly 18.

By automatically opening/closing the valves 21, 22 by said electronic module 8 a flowing (flow direction 23) of said (sample) water 5 could be controlled, i.e. an entering/leaving of said water 5 into/out of said flow block module 18, i.e. the cell body 2, could be controlled.

Said measuring electrode 3 will be cleaned hydrodynamicly by said cell sand 9 (hydrodynamic cell sand cleaning), for example silica sand or corundum, filled in said cell body 2 and grinding the measuring electrode 3 while the measuring electrode 3 is flushed by the (sample) water 5 flowing through the cell body 2.

A cell sand dosage tank 6 is connected to said cell body 2, i.e. said cell sand dosage tank 6 is being fixed at a cell body cover 10 - or can be mounted at a flow block module housing or wall with being connected to said cell body 2, also using an electromagnetic back pressure cell sand dosing valve 7, for automatically dosing cell sand 9 out of said cell sand dosage tank 6 into said cell body 2 - in an exactly defined amount.

Said electromagnetic back pressure cell sand dosing valve 7 is connected to said electronic module 8 - by a wire 13 -, said electronic module 8 further controlling said automatically dosing of cell sand 9 out of said cell sand dosage tank 6 into said cell body 2 - in said exactly defined amount.

A cell sand storage tank 17 - filled with said cell sand 9 - is connected to said cell sand dosing tank 6 providing said cell sand dosing tank 6 with said cell sand 9.

A cell sand drain 11 is connected to said cell body 2 by a cell sand draining means 12, i.e. by an electromagnetic back pressure cell sand draining valve 12, for automatically draining said cell sand 9 out of said cell body 2 in said cell sand drain 11.

The electronic module 8 is also be connected - via a wire 13 - to said electromagnetic back pressure cell sand draining valve 12 for controlling said automatically draining of the cell sand 9 out of said cell body 2.

The cell sand 9 required for the hydrodynamic (cell sand) cleaning of the chlorine sensor measuring electrode 3 grinds itself down over time until the cell sand 9 becomes very fine.

An efficiency of the hydrodynamic cleaning will decrease while the cell sand 9 becomes fine - and, consequently, the (free) chlorine measurement would no longer be reliable.

The cell sand 9 required for the hydrodynamic (cell sand) cleaning of the chlorine sensor measuring electrode 3 also will be flushed out by the (sample) water 5 flowing through the cell body 2 gradually with insufficient cell sand 9 causing insufficient cleaning of the measuring electrode 3 and unreliable (free) chlorine measurement.

Therefore, the cell sand 9 has to be controlled continuously and refilled and/or replaced, for example quarterly or half-yearly.

This cell sand refill/-placement procedure can be performed automatically by said electrode sensor 1 while a respective measuring/controlling software is implemented - as a software program - in a processor of said electronic module 8 - controlling/running said refill/-placement procedure automatically.

An operator starts a display menu item 15 provided - at a display 14 of the electronic module 8 - at the electronic module 8 while said automatic controlling/running of said refill/-placement procedure then works automatically.

The water inlet valve 21 - and in case of the pressurized version - the water outlet valve 22 as well - will close and a sample water supply of the flow block assembly 18 will be stopped.

The cell sand draining valve 12 will open, pressure will release and the cell body 2 with the sample water 5 and the (exhausted) cell sand 9 therein will be emptied.

After the cell body 2 is empty the water inlet valve 21 will open again to flush out a remaining rest of the cell sand 9 and clean the cell body 2.

After flushing out and cleaning the cell body 2 the water inlet valve 21 and the cell sand draining valve 12 are closed.

The cell sand dosing valve 7 will open and the exactly specified amount of (new) cell sand 9 can flow out of the cell sand dosage tank 6 into the cell body 2.

The cell sand dosing valve 7 will be closed and the cell sand dosage tank 6 is filled by the cell sand storage tank 17 with the exactly specified amount of (new) cell sand 9 for a next (maintenance/refill/replacement) cycle again.

The water inlet valve 21 - and in case of the pressurized version - the water outlet valve 22 as well - will open and a sample water supply of the flow block assembly 18 is running.

The automatic cell sand dosage, incl. flushing/cleaning the cell body 2, is completed.

FIG 2 shows said electrode sensor 1 with said backlight (illumination) 20.

FIG 2 shows an installation of a couple of LEDs (six LEDs 27) arranged on both sides - relatively - to said cell body 2 while said LEDs 27 being fixed at a backside outside or backside inside of said flow block assembly 18 and being (electronically) connected to said electronic module 8 by wires 13.

An electric supply for said LEDs 27 is realized by said electronic module 8. The software of the electronic module 8 is expanded by a display menu item "cell body backlight on/off" 16, while said LED backlight 20, i.e. said LEDs 27, can be turned on/off by an operator.

While said backlight 20 is turned on, visuality to/on said cell body 2, i.e. said measuring electrode 3, can be improved and/or a visual check of said hydrodynamic cleaning can be enabled.

## Claims

1. An electrode sensor arrangement (1) comprising
- a cell body (2) with a measurement electrode (3) to be arranged in said cell body (2) for measuring a physical parameter of a fluid (5) flowing through said cell body (2),
- a cell sand dosage tank (6) connected to said cell body (2) using a cell sand dosing means (7) for automatically dosing cell sand (9) out of said cell sand dosage tank (6) into said cell body (2) and
- an electronic device (8) connected to said cell sand dosing means (7) for controlling said automatically dosing of cell sand (9) into said cell body (2).

2. An electrode sensor arrangement (1) according to any preceding claim wherein said measurement electrode (3) is a chlorine electrode (3) measuring a concentration of a chlorine species dissolved in said fluid (5), especially measuring a free chlorine or chlorine dioxide equivalent in said fluid (5).

3. An electrode sensor arrangement (1) according to any preceding claim wherein said physical parameter to be measured is a concentration of a chlorine species dissolved in said fluid (5), a free chlorine or chlorine dioxide equivalent in said fluid (5).

4. An electrode sensor arrangement (1) according to any preceding claim wherein said cell sand dosing means (7) comprises a cell sand dosing valve (7), especially an electromagnetic valve, being controlled by said electronic device (8).

5. An electrode sensor arrangement (1) according to any preceding claim wherein said electronic device (8) is further connected to said measurement electrode (3) equipped for visualising and processing a measurement of said measurement electrode (3) and for controlling said measurement, said visualising and said processing.

6. An electrode sensor arrangement (1) according to any preceding claim wherein said electronic device (8) is an electronic module, especially further connectable to a computer.

7. An electrode sensor arrangement (1) according to any preceding claim further comprising a flow block assembly (18) with said cell body (2) arranged in said flow block assembly (18), said flow block assembly (18) comprising a fluid inlet (4) and a fluid outlet (19) for said fluid (5) flowing into and out of said flow block assembly (18).

8. An electrode sensor arrangement (1) according to the preceding claim further comprising a fluid inlet valve (21), especially an electromagnetic valve (21), wherein said fluid inlet (4) is connected with said fluid inlet valve (21), said fluid inlet valve (21) being connected and controlled by said electronic device (8) for controlling said fluid (5) flowing into said flow block assembly (18).

9. An electrode sensor arrangement (1) according to claim 7 or 8 wherein said flow block assembly (18) is a pressurised flow armature, especially comprising a fluid outlet valve (22), wherein said fluid outlet (19) is connected with said fluid outlet valve (22), said fluid outlet valve (22) being connected and controlled by said electronic device (8) for controlling said fluid (5) flowing out of said flow block assembly (18).

10. An electrode sensor arrangement (1) according to claim 7 or 8 wherein said flow block assembly (18) is a pressureless flow armature, especially comprising a drain (24), wherein said fluid outlet (19) is connected with said drain (24) for said fluid (5) flowing out of said flow block assembly (18).

11. An electrode sensor arrangement (1) according to any preceding claim further comprising a cell sand drain (11) connected to said cell body (2) using a cell sand draining means (12) for automatically draining cell sand (9) out of said cell body (2) in said cell sand drain (11).

12. An electrode sensor arrangement (1) according to the preceding claim said electronic device (8) further being connected to said cell sand draining means (12), especially an electromagnetic valve (12), for controlling said automatically draining of cell sand (9) out of said cell body (2).

13. An electrode sensor arrangement (1) according to any preceding claim further comprising a lightening means (20), especially a back lightening means (20), for lightening, especially for back lightening, said cell body (2).

14. An electrode sensor arrangement (1) according to the preceding claim wherein said lightening means (20) are one LED (27) or more LEDs (27) arranged as said back lightening means (20) at said cell body (2) to improve a view into said cell body (2).

15. An electrode sensor arrangement (1) according to any preceding claim used for an automatic cell sand refill of said electrode sensor arrangement (1) by
- stopping said fluid (5) flowing into said cell body (2),
- controlling said cell sand dosing means (7) for automatically dosing said cell sand (9) out of said cell sand dosage tank (6) into said cell body (2),
- restarting said fluid (5) flowing into said cell body (2).
